# EUROPEAN PATENT APPLICATION

(11) **EP 4 520 426 A1**
(43) Date of publication of application: **12.03.2025**
(21) Application number: 24195366.0
(22) Date of filing: 20.08.2024
(51) Int. Cl.: B01F 33/301, B01F 33/3039, B01F 33/30, B01F 25/433, B01F 33/82, B01F 23/45

(54) **MICROFLUIDIC CHIP**

(30) Priority: 25.08.2023 KR 20230111680
(71) Applicant: Amorepacific Corporation, Seoul 04386 (KR)
(72) Inventor: OH, Soojung, 17074 Yongin-si, Gyeonggi-do (KR); HAN, Kyungsup, 17074 Yongin-si, Gyeonggi-do (KR); LEE, Jeongin, 17074 Yongin-si, Gyeonggi-do (KR); PI, Bongsoo, 17074 Yongin-si, Gyeonggi-do (KR); BAEK, Heungsoo, 17074 Yongin-si, Gyeonggi-do (KR)
(74) Representative: Viering, Jentschura & Partner mbB Patent- und Rechtsanwälte

(57) **Abstract**

A microfluidic chip comprising:

an inlet part (110) in which a first inlet (112) is provided into which a first fluid is injected; and

a middle part (120) in which a first flow path (122) is provided in which the first fluid can flow,

wherein on the first flow path (122), a mixed raw material (M) is pre-stored, and an absorption member (130) is provided in which the first fluid can pass through.

## Description

### TECHNICAL FIELD

The present disclosure relates to a microfluidic chip.

### BACKGROUND

Cosmetics are used for beauty and skin health, and various devices have been proposed to manufacture cosmetics. In particular, a market for customized cosmetics that reflect a user's skin condition and needs is growing, and various devices for manufacturing such customized cosmetics are being proposed.

Further, in order to provide fresh cosmetics to consumers, various attempts are being made to create customized cosmetics on the spot.

As part of these attempts, there was an attempt to manufacture customized cosmetics using a microfluidic chip.

However, in a conventional microfluidic chip, when a fluid is injected into a micro flow path having a complicated design (repetition of twists, intersections, etc.) at different times, or when two or more fluids are used, there was a problem in which flow of the fluid was inhibited by air (air bubbles) on the micro flow path or a problem in which mixing of the fluids was not carried out effectively.

### [Prior art]

Korean Registered Patent Publication No. 10-0222000 (1999 Jun. 30)

### SUMMARY

A microfluidic chip according to an embodiment of the present disclosure was proposed to solve the above problem and aims to produce a well-stirred cosmetic content by mixing fluids (e.g., first fluid and mixed raw material M) after removing the air (air bubbles) inside the microfluidic chip.

According to an embodiment, a microfluidic chip including an inlet part 110 in which a first inlet 112 is provided into which a first fluid is injected; and a middle part 120 in which a first flow path 122 is provided in which the first fluid can flow, wherein on the first flow path 122, a mixed raw material M is pre-stored, and an absorption member 130 is provided in which the first fluid can pass through.

Further, the microfluidic chip may be provided wherein the absorption member 130 includes portions with different densities, and the mixed raw material M is disposed in portions with a relatively high density.

Further, the microfluidic chip may be provided wherein the absorption member 130 includes a first absorption member 132 through which the first fluid can pass through; and a second absorption member 134 in which the mixed raw material M is stored and provided as a denser medium than the first absorption member 132.

Further, the microfluidic chip may be provided wherein when the first flow path 122 is cut in a direction perpendicular to a direction in which the first flow path 122 extends, the absorption member 130 is disposed throughout the entire cross-section of the first flow path 122.

Further, the microfluidic chip may be provided wherein the first fluid flows and penetrates the absorption member 130 on the first flow path 122, and a velocity of the first fluid in the first absorption member 132 is provided to be faster than a velocity of the first fluid in the second absorption member 134.

Further, the microfluidic chip may be provided wherein the first absorption member 132 is provided as a sponge having a relatively sparse area, and the second absorption member 134 is provided as a sponge having a relatively dense area.

The microfluidic chip may be provided wherein on the first flow path 122, a mixed fluid is formed in which the first fluid and the mixed raw material M are mixed, and the microfluidic chip 10 further includes a vortexing part 140 in which a vortex forming flow path 141 configured to promote mixing of the mixed fluid by causing a vortex.

The microfluidic chip may be provided wherein the vortex forming flow path 141 includes a first rotating flow path 1411 which guides the entering mixed fluid to rotate in one direction; a second rotating flow path 1412 which guides the mixed fluid rotating in one direction to rotate in the other direction; and a direction converting flow path 1413 which changes the rotating direction of the mixed fluid between the first rotating flow path 1411 and the second rotating flow path 1412.

Further, the microfluidic chip may be provided wherein the inlet part 110 further includes a second inlet 114 into which a second fluid is injected, and the middle part 120 further includes a second flow path 124 in which the second fluid can flow; and a merging part 129 where the first flow path 122 and the second flow path 124 meet, wherein in the merging part 129 the first fluid, the mixed raw material M, and the second fluid are mixed to create a mixed fluid.

A microfluidic chip according to an embodiment of the present disclosure is configured to mix fluids (first fluid and mixed raw material M) after removing air (air bubbles) inside the microfluidic chip.

Further, it is configured to produce a well-stirred cosmetic content.

### BRIEF DESCRIPTION OF DRAWINGS

FIG. 1 shows a microfluidic chip 10 according to an embodiment of the present disclosure.
FIG. 2 is an enlarged view of a middle part 120 of the microfluidic chip 10 of FIG. 1.
FIG. 3 is an enlarged view of a vortexing part 140 of the microfluidic chip 10 of FIG. 1.
FIG. 4 shows a cross-section taken along an imaginary line A-A' in FIG. 1.
FIG. 5 shows a cross-section taken along an imaginary line B-B' in FIG. 1.
FIG. 6 is a diagram conceptually showing a cosmetic manufacturing apparatus 1 including the microfluidic chip 10 of the present embodiment.

### DETAILED DESCRIPTION OF EXEMPLARY EMBODIMENTS

Hereinafter, specific embodiments of the present disclosure will be described in more detail with reference to the accompanying drawings. In addition, in describing the present disclosure, the detailed description of known configurations or functions incorporated herein will be omitted if it is deemed that it may unnecessarily obscure the gist of the present disclosure.

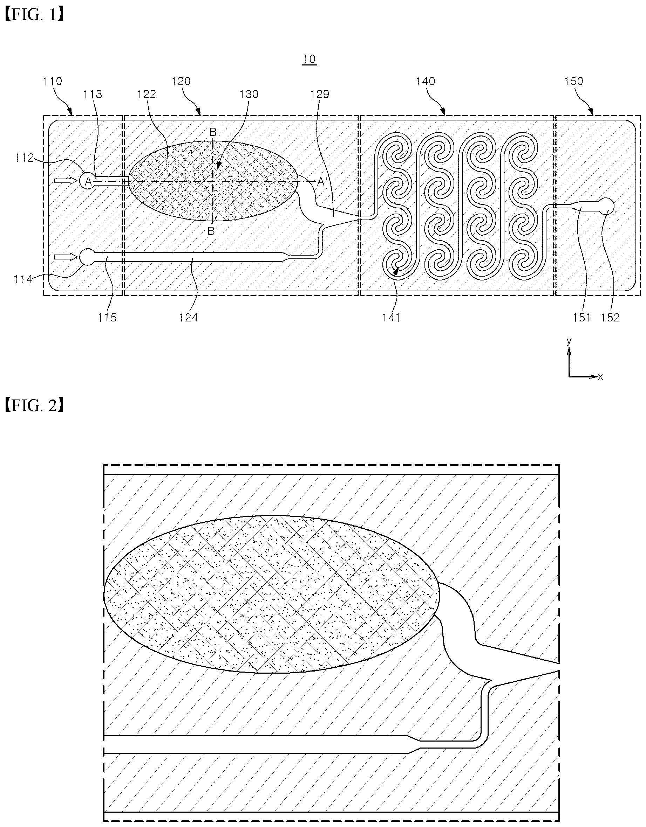

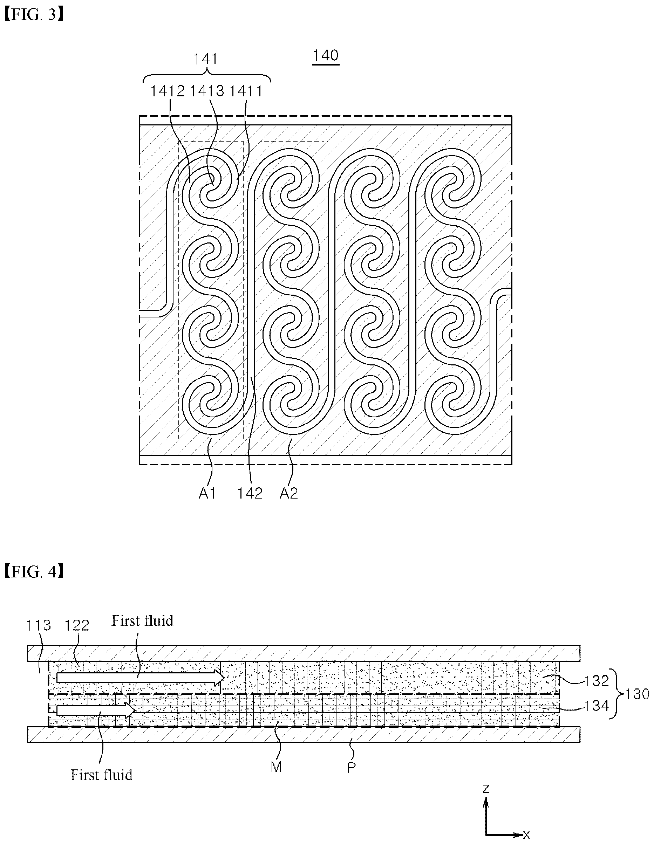

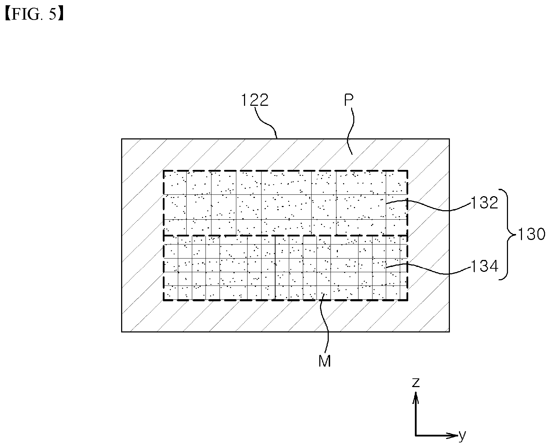

FIG. 1 shows a microfluidic chip 10 according to an embodiment of the present disclosure, FIG. 2 is an enlarged view of a middle part 120 of the microfluidic chip 10 of FIG. 1, FIG. 3 is an enlarged view of a vortexing part 140 of the microfluidic chip 10 of FIG. 1, FIG. 4 shows a cross-section taken along an imaginary line A-A' in FIG. 1, and FIG. 5 shows a cross-section taken along an imaginary line B-B' in FIG. 1.

Referring to FIGS. 1 to 5, the microfluidic chip 10 according to an embodiment of the present disclosure may include an inlet part 110 in which a first inlet 112 is provided into which a first fluid is injected; and a middle part 120 in which a first flow path 122 through which the first fluid may flow is provided, wherein a mixed raw material M is pre-stored on the first flow path 122, and an absorption member 130 may be provided in which the first fluid may pass though.

Further, the microfluidic chip 10 may further include a vortexing part 140 extending from the middle part 120; and a dispensing part 150 extending from the vortexing part 140.

In the present embodiment, the absorption member 130 provided with the mixed raw material M may be disposed on the middle part 120.

Further, the absorption member 130 may include a first absorption member 132 provided as a relatively sparse medium; and a second absorption member 134 provided as a relatively dense medium, and the mixed raw material M may be absorbed and stored on the second absorption member 134.

Likewise, the absorption member 130 may include portions with different densities (e.g., first absorption member 132, second absorption member 134), and the mixed raw material M may be disposed in portions with a relatively high density (e.g., second absorption member 134).

Accordingly, the first fluid may first pass through the first absorption member 132 which has a relatively lower density, and push the air (air bubbles) on the first absorption member 132 to downstream of the first absorption member 132.

In this case, the first fluid and the mixed raw material M are mixed after the air (or air bubbles) in the absorption member 130 is removed. Therefore, the mixing of the first fluid and the mixed raw material M can be readily carried out.

In the present embodiment, more than one fluid may be injected into the microfluidic chip 10 from outside.

The microfluidic chip 10 of the present embodiment may be understood as a device which creates a cosmetic content by mixing the mixed raw material M pre-stored inside the microfluidic chip 10 and the first fluid (or first fluid, second fluid) injected from outside of the microfluidic chip 10.

In the present embodiment, two types of fluids (first fluid, second fluid) being introduced into the microfluidic chip 10 will be described as an example. However, the idea of the present disclosure is not limited thereto, and if only one type of fluid (e.g., first fluid) is injected into the microfluidic chip 10, a second inlet 114 and a second flow path 124 connected thereto may not be provided.

In the present embodiment, the mixed raw material M may be provided in a form of a fluid, a powder that dissolves in the first fluid, or a gel state that dissolves in the first fluid. In the present embodiment, the mixed raw material M is provided as a viscous fluid as an example.

In the present embodiment, various cosmetic contents (e.g., emulsion) may be created according to the type of the mixed raw material M pre-stored in the microfluidic chip 10.

In the present embodiment, one type of the mixed raw material M is stored in microfluidic chip 10 as an example.

The microfluidic chip 10 may be provided interchangeably with a cosmetic manufacturing apparatus 1 which will be described later. For example, the microfluidic chip 10 may be provided for sale separately, and different types of cosmetic contents (e.g., emulsion) may be created by using only the microfluidic chip 10 interchangeably.

Further, the mixed raw material M having various ingredients may be stored in the microfluidic chip 10 so that various types of cosmetic contents (e.g., emulsion) may be created according to a user's preference.

In the present disclosure, the cosmetic content may include an emulsion in which at least one of the first fluid, the second fluid, and the mixed raw material M is mixed and emulsified; a lotion in which at least one of the first fluid, the second fluid, and the mixed raw material M is solubilized; and mixed water in which at least one of the first fluid, the second fluid, and the mixed raw material M is simply mixed.

In the present embodiment, the cosmetic content dispensed from microfluidic chip 10 may be a solubilized lotion. For example, the solubilized lotion, a skin lotion, an essence, a perfume, etc. may be included.

In the present embodiment, the cosmetic content dispensed from the microfluidic chip 10 may be the emulsion. In this case, the cosmetic content may include an O/W (Oil in Water) emulsion which is manufactured by uniformly dispersing a hydrophobic fluid such as oil into small particles in a hydrophilic fluid such as water, or a W/O (Water in Oil) emulsion which is manufactured by uniformly dispersing a hydrophilic fluid into small particles in a hydrophobic fluid.

For example, if the cosmetic content is provided as the emulsion, the first fluid may be provided as a hydrophilic fluid, and the mixed raw material M may be provided as a hydrophobic fluid. Alternatively, the first fluid may be provided as the hydrophobic fluid, and the mixed raw material M may be provided as the hydrophilic fluid.

The microfluidic chip 10 of the present embodiment may be understood as a configuration in which a micro flow path is formed inside a plate P which is provided in a flat plate shape.

Here, the micro flow path may include a first injection flow path 113, and a second inlet 114 formed in the inlet part 110; a first flow path 122 formed in the middle part 120; a vortex forming flow path 141 formed in the vortexing part 140; and a discharge flow path 151 formed in the dispensing part 150.

A cross-section of the micro flow path formed inside the microfluidic chip 10 may be circular or rectangular. A diameter of the micro flow path may be 0.5mm to 1mm, preferably 0.6mm to 0.8mm.

As such, by forming the micro flow path inside the microfluidic chip 10, the efficiency of mixing or emulsifying fluids may be increased by increasing the flow rate of the fluid flowing along the micro flow path.

Meanwhile, in the present embodiment, the micro flow paths formed in the microfluidic chip 10 may form a monolayer passage. Here, the monolayer passage may be understood as the micro flow path being disposed on a same plane (xy-plane).

Hereinafter, introduction of two types of fluids (first fluid and second fluid) into the microfluidic chip 10 will be explained as an example.

The microfluidic chip 10 may include the inlet part 110, the middle part 120, the vortexing part 140, and the dispensing part 150.

The inlet part 110 may include the first inlet 112 into which the first fluid is injected; and the second inlet 114 into which the second fluid is injected.

Here, the first fluid and the second fluid may be provided as different types of fluids. However, the idea of the present disclosure is not limited thereto, and the first fluid and second fluid may be provided as a same type of fluid.

The inlet part 110 may include the first injection flow path 113 extending from the first inlet 112; and a second injection flow path 115 extending from the second inlet 114.

The first injection flow path 113 of the inlet part 110 may be connected to the first flow path 122 of the middle part 120, and the second injection flow path 115 of the inlet part 110 may be connected to the second flow path 124 of the middle part 120.

The middle part 120 may include the first flow path 122 in which the first fluid may flow.

On the first flow path 122, the absorption member 130 may be provided in which the mixed raw material M is pre-stored.

For example, the absorption member 130 may be provided as a sponge having a dense area (e.g., second absorption member 134) and a sparse area (e.g., first absorption member 132). That is, the absorption member 130 includes portions with different densities, and the mixed raw material M may be disposed in portions with a relatively high density.

Specifically, the absorption member 130 may include the first absorption member 132 through which the first fluid may pass; and the second absorption member 134 in which the mixed raw material M is stored and provided as a denser medium than the first absorption member 132.

The second absorption member 134 may be disposed at a lower side than the first absorption member 132. Here, the lower side may be understood as a direction in which gravity acts (direction in which the mixed raw material M is disposed) on the first fluid when the first fluid passes through the absorption member 130. With respect to FIG. 4, the lower side may be understood as a -z-axis direction.

The first fluid may pass through both the first absorption member 132 and the second absorption member 134.

However, because the first absorption member 132 is provided as a less dense material than the second absorption member 134, the first fluid may flow relatively faster in the first absorption member 132 than in the second absorption member 134.

The first fluid may first pass through the first absorption member 132 and then through the second absorption member 134.

First, the first fluid may push air (air bubbles) to the outside of the first absorption member 132 while passing through the first absorption member 132.

Afterwards, the first fluid may pass through the second absorption member 134 and push the mixed raw material M which is absorbed in the second absorption member 134, to the outside of the second absorption member 134. Accordingly, the mixed raw material M and the first fluid may be mixed to create a mixed fluid.

The absorption member 130 may be disposed on a passage of the first flow path 122 in which the first fluid passes.

Specifically, when the first flow path 122 is cut in a direction perpendicular (B-B') to a direction in which the first flow path 122 extends, the absorption member 130 may be disposed throughout the entire cross-section of the first flow path 122. That is, the first fluid is provided so that it must pass through the absorption member 130.

When the absorption member 130 is viewed from the top (xy plane), the absorption member 130 may be formed into an oval shape with a major axis in the direction in which the first flow path 122 extends (refer to FIG. 2).

When the first flow path 122 is cut in the direction perpendicular (B-B') to the direction in which the first flow path 122 extends, the first absorption member 132 may be disposed on the upper side, and the second absorption member 134 may be placed on the lower side (refer to FIG. 5).

Further, when the first flow path 122 is cut in a direction in which the first flow path 122 extends (A-A'), the first absorption member 132 may be disposed on the upper side and the second absorption member 134 may be disposed on the lower side (refer to FIG. 4) in the same manner.

Further, a length of the absorption member 130 measured in the direction in which the first flow path 122 extends (A-A') may be longer than a length of the absorption member 130 measured in the direction perpendicular (B-B') to the direction in which the first flow path 122 extends.

In the present embodiment, the length of the absorption member 130 measured in the direction in which the first flow path 122 extends (A-A') will be expressed as a major axis length, and the length of absorption member 130 measured in the direction perpendicular (B-B') to the direction in which the first flow path 122 extends will be expressed as a minor axis length.

In the present embodiment, the major axis length of the absorption member 130 may be formed to be longer than the minor axis length. For example, the major axis length of the absorption member 130 may be provided as two to three times the minor axis length of the absorption member 130.

Further, the area of the absorption member 130 may be provided as more than 1/2 of the area of the entire micro flow path of the microfluidic chip.

In the present embodiment, the first injection flow path 113, the second injection flow path 115, the second flow path 124, the vortex forming flow path 141, and the discharge flow path 151 may be provided as a micro flow path with a diameter of 0.5 mm to 1 mm, and the first flow path 122 in which the absorption member 130 is disposed may be provided as a flow path with a diameter exceeding 1 mm. In other words, remaining flow paths excluding the first flow path 122 from the entire flow paths of the microfluidic chip 10 may be expressed as the micro flow paths, and the first flow path 122 may be expressed as a general flow path.

For example, an average diameter of the first flow path 122 in which the absorption member 130 is disposed may be provided as more than 10 times that of the vortex forming flow path 141. In this case, by removing the air (air bubbles) in the absorption member 130 and forming a vortex in the fluid in the vortex forming flow path 141, stirring of the cosmetic content may be carried out effectively.

While the first fluid moves through the first absorption member 132, the first fluid may push out the air (air bubbles) in the first absorption member 132 and then be mixed with the mixed raw material M stored in the second absorption member 134.

At this time, the mixed raw material M stored in the second absorption member 134 may rise to the upper side and be mixed with the first fluid, and may be pushed downstream and be mixed with the first fluid.

Here, the downstream may be understood as a position disposed relatively behind with respect to the direction in which the first fluid flows. For example, the vortex forming flow path 141 described later may be understood as a configuration disposed at downstream of the first injection flow path 113. On the contrary, upstream may be understood as a position disposed relatively forward with respect to the direction in which the first fluid flows. For example, the first injection flow path 113 may be understood as a configuration disposed at the upstream of the vortex forming flow path 141.

In the present embodiment, if the mixed raw material M is provided as a fluid, viscosity of the mixed raw material M may be provided to be less than viscosity of the first fluid.

In this case, the mixed raw material M may be stored in the second absorption member 134 while the first fluid flows through the first absorption member 132, and may be pushed downstream by the first fluid when the first fluid passes through the second absorption member 134.

Further, in the middle part 120, the second flow path 124 may be provided through which the second fluid injected from the second inlet 114 may flow.

In the present embodiment, the absorption member 130 is illustrated to be not provided on the second flow path 124, but the above-described absorption member may also be provided on the second flow path 124. In this case, the absorption member provided on the second flow path 124 may be provided with a raw material that may be mixed with the second fluid as a raw material different from the above-described mixed raw material M.

In the middle part 120, a mixed fluid may be created as the first fluid and the mixed raw material M are mixed.

Further, the middle part 120 may include a merging part 129 where the mixed fluid and the second fluid meet.

The second flow path 124 and the first flow path 122 may be connected in the merging part 129.

An average diameter of the second flow path 124 may be provided to be smaller than the average diameter of the first flow path 122. In this case, the first fluid and the mixed raw material M may be more easily mixed by the flow rate of the second fluid introduced into the merging part 129.

In the present embodiment, when the first fluid is introduced into the microfluidic chip 10, the mixed fluid may be understood as a fluid that is a mixture of the first fluid and the mixed raw material M. Further, when the first fluid and second fluid are introduced into the microfluidic chip 10, the mixed fluid may be understood as a fluid that is a mixture of the first fluid, the second fluid, and the mixed raw material M.

The middle part 120 may include the second flow path 124 through which the second fluid may flow; and the merging part 129 where the first flow path 122 and the second flow path 124 may meet. In the merging part 129, the first fluid, the mixed raw material M, and the second fluid may be mixed to create a mixed fluid.

The vortexing part 140 may include the vortex forming flow path 141 which may promote mixing of the mixed fluid by creating a vortex. Here, the vortex forming flow path 141 may extend from the merging part 129.

The vortexing part 140 may include a plurality of the vortex forming flow paths 141 that generates a vortex by changing a direction of flow of the fluid (or mixed fluid).

For example, if the cosmetic content is provided as an emulsion, the mixed fluid may be broken into particles by the vortex generated in the vortex forming flow path 141, and an emulsion including emulsified particles may be generated whose size gradually decreases as they travel downstream. For example, emulsified particles flowing through a second area A2 of the vortexing part 140 may be provided to be smaller than emulsified particles flowing through a first area A1.

Here, emulsification may be understood as a technique of dispersing one of two immiscible fluids, such as water and oil, into small particles and disposing them in a stable state within the other liquid.

The vortex forming flow path 141 may be formed continuously in plural from the vortexing part 140 to the first area A1.

Further, the vortex forming flow path 141 may be formed continuously in plural in the second area A2 disposed at downstream of the first area A1.

The vortex forming flow path 141 formed in the first area A1 and the vortex forming flow path 141 formed in the second area A2 may be connected through a connection flow path 142, and the connection flow path 142 may be formed as a straight line shaped micro flow path.

In the present embodiment, the vortex forming flow path 141 will be described to be configured to rotate an entering fluid in one direction (clockwise in the present embodiment) and then rotate it in the other direction (counterclockwise in the present embodiment) as an example.

Specifically, each vortex forming flow path 141 may include a first rotating flow path 1411 which guides the entering mixed fluid to rotate in one direction; a second rotating flow path 1412 which guides the mixed fluid rotating in one direction to rotate in the other direction; and a direction converting flow path 1413 which changes the rotating direction of the mixed fluid between the first rotating flow path 1411 and the second rotating flow path 1412.

If the cosmetic content is provided as an emulsion, then an emulsion may be formed in which a dispersed-phase fluid broken into small particles by passing through the vortex forming flow path 141 is stably present in an external-phase fluid.

The dispensing part 150 may extend from the vortexing part 140.

The dispensing part 150 may include the discharge flow path 151 extending from the vortex forming flow path 141 of the vortexing part 140; and a discharge hole 152 from which generated cosmetic content is dispensed.

The discharge flow path 151 may be formed in a shape bent by a preset angle (e.g., 90 degrees) from the connection flow path 142 and then extended in one direction.

The discharge flow path 151 extends in a direction from the vortex forming flow path 141 to the discharge hole 152 and may include a portion having an increasing width.

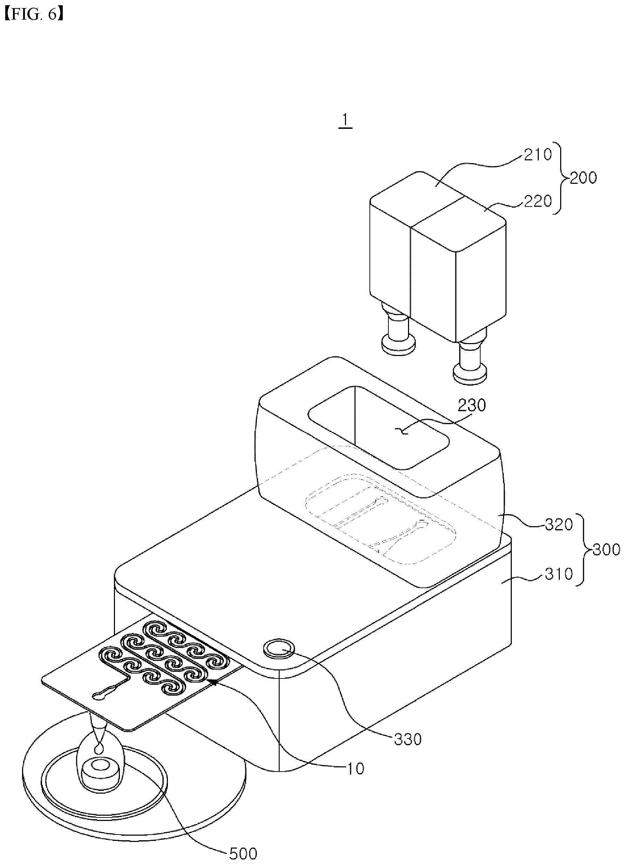

FIG. 6 is a diagram conceptually showing a cosmetic manufacturing apparatus 1 including the microfluidic chip 10 of the present embodiment.

Referring to FIG. 6, the cosmetic manufacturing apparatus 1 may include a fluid storage part 200 configured to store the first fluid and the second fluid; the above-described microfluidic chip 10 configured to receive at least one of the first fluid and the second fluid from the fluid storage part 200; and a body 300 which accommodates the fluid storage part 200 and the microfluidic chip 10.

Here, the microfluidic chip 10 may be provided interchangeably with the body 300.

The fluid storage part 200 may include a first storage part 210 which stores the first fluid; and a second storage part 220 which stores the second fluid. Capacities of the first storage part 210 and the second storage part 220 may be the capacities in which the first fluid and the second fluid are dispensed in a single operation, respectively. However, the idea of the present disclosure is not limited thereto, and the capacities of the first storage part 210 and the second storage part 220 may be provided as capacities in which the first fluid and the second fluid are dispensed through multiple operations, respectively.

Further, the first storage part 210 and the second storage part 220 may be provided interchangeably with the body 300.

The body 300 may include a first body 310 in which a first insertion hole (not shown) is formed which provides a space for insertion of the microfluidic chip 10; and a second body 320 to which a second insertion hole 230 is formed which provides a space for insertion of the fluid storage part 200.

In the present embodiment, the first body 310 and the second body 320 are described separately, but the first body 310 and the second body 320 may be a single member formed integrally.

On one side of the body 300, an operation button 330 may be provided to execute an operation for generating a cosmetic content.

When the operation button 330 is pressed, the fluid stored in the fluid storage part 200 is moved to the microfluidic chip 10 and then mixed and emulsified with the mixed raw material M pre-stored in the microfluidic chip 10 to generate a cosmetic content (e.g., emulsion), which may be dispensed to the outside of the microfluidic chip 10.

Further, in the first insertion hole of the body 300, a recognition part (not shown) configured to recognize types of the microfluidic chip 10 may be provided.

When the type of the microfluidic chip 10 is recognized by the recognition part (not shown), a driving part (not shown) controlled by a control part (not shown) may apply a pressure corresponding to the type of the microfluidic chip 10 to the micro flow path of the microfluidic chip 10.

Additionally, the above-described microfluidic chip 10 may be provided separably from the body 300.

The microfluidic chip 10 may accommodate the first fluid from the first storage part 210 and the second fluid from the second storage part 220.

Hereinafter, an operation process of the cosmetic manufacturing apparatus 1 will be described.

An operation process of the cosmetic manufacturing apparatus 1 according to the present embodiment may include inserting a microfluidic chip 10 into a body 300; recognizing types of the microfluidic chip 10 by a recognition part (not shown); creating a mixed fluid by a first fluid pushing air on a micro flow path, and the first fluid and a mixed raw material M are mixed, as a preset pressure according to types of the microfluidic chip 10 is applied on the micro flow path of the microfluidic chip 10 by a driving unit; and creating a cosmetic content as the mixed fluid travels along a vortex forming flow path 141 formed in a vortexing part 140; and discharging the cosmetic content to the outside of the microfluidic chip 10.

The cosmetic content discharged to the outside of the microfluidic chip 10 may be contained in a cosmetic container 500 disposed at a lower part of a discharge hole 152.

Although the microfluidic chip 10 according to an embodiment of the present disclosure has been described as specific embodiments, they are only examples, and the present disclosure is not limited thereto, and should be interpreted as having the widest range according to the basic idea disclosed in this specification. A person of ordinary skill in the art may combine and substitute any of the disclosed embodiments to practice embodiments not shown, without departing from the scope of the present disclosure to scope of right. In addition, it is apparent that a person of ordinary skill in the art can be readily changed or modified to facilitate the embodiments disclosed herein, and that such changes or modifications also fall within the scope of the present disclosure to scope of right.

## Claims

1. A microfluidic chip comprising:
an inlet part (110) in which a first inlet (112) is provided into which a first fluid is injected; and
a middle part (120) in which a first flow path (122) is provided in which the first fluid can flow,
wherein on the first flow path (122), a mixed raw material (M) is pre-stored, and an absorption member (130) is provided in which the first fluid can pass through.

2. The microfluidic chip of claim 1,
wherein the absorption member (130) comprises portions with different densities, and the mixed raw material (M) is disposed in portions with a relatively high density.

3. The microfluidic chip of claim 1,
wherein the absorption member (130) comprises
a first absorption member (132) through which the first fluid can pass through; and
a second absorption member (134) in which the mixed raw material (M) is stored and provided as a denser medium than the first absorption member (132).

4. The microfluidic chip of claim 1,
wherein when the first flow path (122) is cut in a direction perpendicular to a direction in which the first flow path (122) extends, the absorption member (130) is disposed throughout the entire cross-section of the first flow path (122).

5. The microfluidic chip of claim 3,
wherein the first fluid flows and penetrates the absorption member (130) on the first flow path (122), and
a velocity of the first fluid in the first absorption member (132) is provided to be faster than a velocity of the first fluid in the second absorption member (134).

6. The microfluidic chip of claim 3,
wherein the first absorption member (132) is provided as a sponge having a relatively sparse area, and
the second absorption member (134) is provided as a sponge having a relatively dense area.

7. The microfluidic chip of claim 1,
wherein on the first flow path (122), a mixed fluid is formed in which the first fluid and the mixed raw material M are mixed, and
the microfluidic chip (10) further comprises
a vortexing part (140) in which a vortex forming flow path (141) configured to promote mixing of the mixed fluid by causing a vortex.

8. The microfluidic chip of claim 7,
wherein the vortex forming flow path (141) comprises
a first rotating flow path (1411) which guides the entering mixed fluid to rotate in one direction;
a second rotating flow path (1412) which guides the mixed fluid rotating in one direction to rotate in the other direction; and
a direction converting flow path (1413) which changes the rotating direction of the mixed fluid between the first rotating flow path (1411) and the second rotating flow path (1412).

9. The microfluidic chip of claim 1,
wherein the inlet part (110) further comprises a second inlet (114) into which a second fluid is injected, and
the middle part (120) further comprises a second flow path (124) in which the second fluid can flow; and
a merging part (129) where the first flow path (122) and the second flow path (124) meet,
wherein in the merging part (129) the first fluid, the mixed raw material (M), and the second fluid are mixed to create a mixed fluid.
